# EUROPEAN PATENT APPLICATION

(11) **EP 2 759 584 A1**
(43) Date of publication of application: **30.07.2014**
(21) Application number: 13152791.3
(22) Date of filing: 25.01.2013
(51) Int. Cl.: C09K 8/58, A61K 8/34, B01J 13/16

(54) **A solid particles-stabilized emulsion and process for preparing the same**

(71) Applicant: Wintershall Holding GmbH, 34119 Kassel (DE)
(72) Inventor: Kimura, Riichiro, 67059 Ludwigshafen (DE); Maurer, Stefan, 67061 Ludwigshafen (DE); Parvulescu, Andrei-Nicolae, 69117 Heidelberg (DE); Siggel, Lorenz, 69121 Heidelberg (DE); Müller, Ulrich, 67435 Neustadt (DE); Frechen, Thomas, 69120 Heidelberg (DE); Hinrichsen, Bernd, 70569 Stuttgart (DE)
(74) Representative: Dierkes, Thorsten

(57) **Abstract**

The presently claimed invention is directed a solid particles-stabilized emulsion containing a continuous phase and a dispersed phase comprising a) water, b) oil and c) at least one layered double hydroxide of general formula (I), wherein water is the continuous phase and oil is the dispersed phase, and a process for preparing the same.

## Description

The presently claimed invention is directed a solid particles-stabilized emulsion containing a continuous phase and a dispersed phase comprising a) water, b) oil and c) at least one layered double hydroxide of general formula (I), wherein water is the continuous phase and oil is the dispersed phase, and a process for preparing the same.

Emulsions are known in the art and are commonly referred to as oil-in-water or water-in-oil emulsions. Emulsions generally have a limited stability, i.e. limited storage life time or shelf life time, and segregate or separate upon prolonged storage, and/or show rapid droplet growth or droplet size increase.

Oil-in-water emulsions have become important in the petroleum industry as a displacing fluid for enhanced oil recovery. When used as a displacing fluid, an emulsion is pumped into a wellbore and displaces oil in subterranean formations. However, an alternative approach to increase the amount of extracted oil would be to form an emulsion in situ in the subterranean formation. These emulsions should have a low viscosity and show high stability even at elevated temperatures in order to allow for easy recovery from the subterranean formation by pumping.

Thus, there is a need in the art to provide emulsion that show high stability, even at higher temperatures such as temperatures in the range of 30-200 °C.

Hence, it is one object of the presently claimed invention to provide emulsions that show a high stability, even at higher temperatures such as temperatures in the range of 30-200 °C.

The object is solved by providing a solid particles-stabilized emulsion containing a continuous phase and a dispersed phase comprising
a) water,
b) oil and
c) at least one layered double hydroxide of general formula (I)

   [M^{II}₍₁₋ₓ₎M^{III}ₓ(OH)₂]^{x+}[Aⁿ⁻] .y H₂O (I),

   wherein
   - M^{II}: denotes a divalent metal ion or 2Li,
   - M^{III}: denotes a trivalent metal ion,
   - Aⁿ⁻: denotes an n-valent anion,
   - n: is 1 or 2,
   - x: is the mole fraction having a value ranging from 0.1 to 0.5 and
   - y: is a value ranging from 0 to 5.0,
which is present in the form of solid particles,
wherein water is the continuous phase and oil is the dispersed phase, whereby oil is present in the form of droplets.

The term "stability" refers to the period up to incipient separation, and in which the emulsion does not visually show segregation, such as the formation of a visible bottom layer of water and/or a visible top layer of oil.

It is noted that WO 2009/87199 A1 discloses emulsions that contain oil, water and solid particles. However, these emulsions require the presence of surfactants in order to achieve sufficient stability of the emulsion. The use of surfactants is usually costly, because they cannot be recovered from the emulsion and subsequently be used again. Therefore, it would be very much appreciated if emulsions were provided that do not contain surfactants so that the solid particles can be recovered without any difficulty.

Hence, it is another object of the presently claimed invention to provide emulsions that show a high stability, even at higher temperatures such as temperatures in the range of 30-200 °C, which are free of surfactants.

For evaluating the stability, as used in this invention, a test method is to be used wherein a sample of 100 g of emulsion is stored in a test tube with an inner diameter of 2.5 cm and sufficient length. The tube is stored at a selected temperature and monitored over time for separation to occur, i.e. for formation of a top or bottom layer. The stability is then the time elapsing between filling the test tube and the observation of the separation phenomenon. The temperature is to be chosen such that it is above the melting temperature of the compound in the emulsions with the highest melting temperature, and below the boiling temperature of the lowest boiling compound of the emulsion. Suitably it is chosen between 30 °C and 200 °C.

An emulsion is a heterogeneous liquid system involving two immiscible phases, with one of the phases being intimately dispersed in the form of droplets in the second phase. The matrix of an emulsion is called the external or continuous phase, while the portion of the emulsion that is in the form of droplets is called the internal, dispersed or discontinuous phase.

A solid particles-stabilized emulsion according to the present invention is an emulsion that is stabilized by solid particles which adsorb onto the interface between two phases, for example an oil phase and a water phase.

Preferably the solid particles-stabilized emulsion comprises 10.0 to 90.0 % by weight water, 10.0 to 90.0 % by weight oil and 0.01 to 10.0 % by weight of at least one layered double hydroxide of general formula (I), more preferably 50.0 to 90.0 % by weight water, 10.0 to 50.0 % by weight oil and 0.01 to 5.0 % by weight of at least one layered double hydroxide of general formula (I), most preferably 70.0 to 90.0 % by weight water, 10.0 to 30.0 % by weight oil and 0.01 to 2.5 % by weight of at least one layered double hydroxide of general formula (I), in each case related to the overall weight of the emulsion. Even more preferably the solid particles-stabilized emulsion comprises 70.0 to 90.0 % by weight water, 10.0 to 30.0 % by weight oil and 0.01 to 1.0 % by weight of at least one layered double hydroxide of general formula (I), related to the overall weight of the emulsion.

"Oil" means a fluid containing a mixture of condensable hydrocarbons.

"Hydrocarbons" are organic material with molecular structures containing carbon and hydrogen. Hydrocarbons may also include other elements, such as, but not limited to, halogens, metallic elements, nitrogen, oxygen, and/or sulfur.

Preferably the oils or hydrocarbons are selected from the group consisting of crude oil, straight and branched chain hydrocarbons having from 7 to 40 carbon atoms such as dodecane, isododecane, squalane, cholesterol, hydrogenated polyisobutylene, isodo-decosane, hexadecane; C₁-C₃₀ alcohol esters of C₁-C₃₀ carboxylic acids and of C₁-C₃₀ dicarboxylic acids such as isononyl isononanoate, methyl isostearate, ethyl isostearate, diisoproyl sebacate, diisopropyl adipate, isopropyl myristate, isopropyl palmitate, methyl palmitate, myristyl propionate, 2-ethylhexyl palmitate, isodecyl neopentanoate, di(2-ethylhexyl) maleate, cetyl palmitate, cetyl stearate, methyl stearate, isopropyl stearate, and behenyl behenate; mono-, di-, and tri-glycerides of C₁-C₃₀ carboxylic acids such as caprylic/capric triglyceride, PEG-6 caprylic/capric triglyceride, and PEG-8 caprylic/capric triglyceride; alkylene glycol esters of C₁-C₃₀ carboxylic acids including ethylene glycol mono- and di-esters of C₁-C₃₀ carboxylic acids and propylene glycol mono- and di-esters of C₁-C₃₀ carboxylic acids such as ethylene glycol distearate; C₁-C₃₀ mono- and poly- esters of sugars and related materials such as glucose tetraoleate; and organopolysiloxane oils such as polyalkyl siloxanes, cyclic polyalkyl siloxanes, and polyalkylaryl siloxanes. It is also contemplated to use propoxylated or ethoxylated forms of the above-exemplified oils. It is further envisaged to use two or more oils as the oil component in the emulsion of the invention.

More preferably the oil is crude oil.

"Crude oil" is defined as a mixture of hydrocarbons that existed in liquid phase in underground reservoirs and remains liquid at atmospheric pressure after passing through surface separating facilities and which has not been processed through a crude oil distillation tower.

Most preferably the oil is crude oil having an API gravity in the range between 20 °API and 40 °API. Such oils, by nature of their composition, usually contain asphaltenes and polar hydrocarbons.

Most preferably the crude oil is crude oil having a a viscosity in the range of 1 to 5000 mPa.s, more preferably in the range of 10 to 1000 mPa.s, most preferably in the range of 25 to 500 mPa.s, each at a temperature of 20 °C according to DIN 53019.

Surprisingly, it was found that the presence of at least one layered double hydroxide of general formula (I) significantly increases the stability of the inventively claimed emulsions.

Layered double hydroxides of general formula (I) (LDH) comprise an unusual class of layered materials with positively charged layers and charge balancing anions located in the interlayer region. This is unusual in solid state chemistry: many more families of materials have negatively charged layers and cations in the interlayer spaces (e.g. kaolinite, Al₂Si₂O₅(OH)₄).

Preferably the at least one layered double hydroxide is represented by the general formula (I)

[M^{II}₍₁₋ₓ₎M^{III}ₓ(OH)₂]^{x+}[Aⁿ⁻] .y H₂O (I),

wherein
M^{II} denotes a divalent metal ion selected from the group consisting of Ca, Mg, Fe, Ni, Zn, Co, Cu and Mn or 2Li,
M^{III} denotes a trivalent metal ion selected from the group consisting of Al, Fe, Cr and Mn,
Aⁿ⁻ denotes an n-valent anion selected from the group consisting of Cl⁻, Br⁻, NO₃-, CO₃²-, SO₄²⁻ and SeO₄²⁻,
x is the mole fraction having a value ranging from 0.1 to 0.5 and
y is a value ranging from 0 to 5.0.

More preferably the at least one layered double hydroxide is represented by the general formula (I)

[M^{II}₍₁₋ₓ₎M^{III}ₓ(OH)₂]^{x+}[Aⁿ⁻] .y H₂O (I),

wherein
M^{II} denotes Mg,
M^{III} denotes a trivalent metal ion selected from the group consisting of Al and Fe, Aⁿ⁻ denotes an n-valent anion selected from the group consisting of Cl⁻, Br⁻, NO₃-, CO₃²⁻, SO₄²⁻ and SeO₄²⁻,
x is the mole fraction having a value ranging from 0.1 to 0.5 and
y is a value ranging from 0 to 5.0.

Preferably x is the mole fraction having a value ranging from 0.2 to 0.33.

Examples of the at least one layered double hydroxide of general formula (I) include hydrotalcite [Mg₆Al₂(CO₃)(OH)₁₆·4(H₂O)], manasseite [Mg₆Al₂(CO₃)(OH)₁₆·4(H₂O)], pyroaurite [Mg₆Fe₂(CO₃)(OH)₁₆·4.5(H₂O)], sjoegrenite [Mg₆Fe₂(CO₃)(OH)₁₆·4.5(H₂O)], stichtite [Mg₆Cr₂(CO₃)(OH)₁₆·4(H₂O)], barbertonite [Mg₆Cr₂(CO₃)(OH)₁₆·4(H₂O)], takovite, reevesite [Ni₆Fe₂(CO₃)(OH)₁₆·4(H₂O)], desautelsite [Mg₆Mn₂(CO₃)(OH)₁₆CO₃·4(H₂O)], motukoreaite, wermlandite, meixnerite, coalingite, chlormagaluminite, carrboydite, honessite, woodwardite, iowaite, hydrohonessite and mountkeithite. More preferably the at least one layered double hydroxide of general formula (I) is selected from the group consisting of hydrotalcite [Mg₆Al₂(CO₃)(OH)₁₆·4(H₂O)], manasseite [Mg₆Al₂(CO₃)(OH)₁₆·4(H₂O)], pyroaurite [Mg₆Fe₂(CO₃)(OH)₁₆·4.5(H₂O)], sjoegrenite [Mg₆Fe₂(CO₃)(OH)₁₆·4.5(H₂O)], stichtite [Mg₆Cr₂(CO₃)(OH)₁₆·4(H₂O)], barbertonite [Mg₆Cr₂(CO₃)(OH)₁₆·4(H₂O)], takovite, reevesite [Ni₆Fe₂(CO₃)(OH)₁₆·4(H₂O)] and desautelsite [Mg₆Mn₂(CO₃)(OH)₁₆CO₃·4(H₂O)]. More preferably the at least one layered double hydroxide is selected from the group consisting of hydrotalcite [Mg₆Al₂(CO₃)(OH)₁₆·4(H₂O)], manasseite [Mg₆Al₂(CO₃)(OH)₁₆·4(H₂O)], pyroaurite [Mg₆Fe₂(CO₃)(OH)₁₆·4.5(H₂O)] and sjoegrenite [Mg₆Fe₂(CO₃)(OH)₁₆·4.5(H₂O)].

The solid particles are made of layered double hydroxide of general formula (I). The actual average particle size should be sufficiently small to provide adequate surface area coverage of the internal oil phase. Preferably the solid particles have an average particle size in the range of 30 nm to 10 µm, more preferably in the range of 30 nm to 2 µm and more most preferably in the range of 50 nm to 100 nm, determined according to SEM images (as defined under Method A).

Preferably, the aspect ratio of the solid particles which are made of layered double hydroxide of general formula (I) is in the range of 1 to 30, more preferably in the range of 1 to 20, most preferably in the range of 1 to 10, even more preferably in the range of 2 to 8, whereby the aspect ratio is defined as diameter/thickness. The aspect ratio, diameter and the thickness are determined according to SEM images (as defined under Method A).

Preferably, the solid particles have a BET surface area in the range of 50 to 400 m²/g, more preferably in the range of 80 to 130 m²/g, according to DIN 66315 at 77 K.

Preferably, the solid particles remain undissolved in the water phase under the inventively used conditions, but have appropriate charge distribution for stabilizing the interface between the internal droplet phase, i.e. oil, and the external continuous phase, i.e. water, to make a solid particles-stabilized oil-in-water emulsion.

Preferably, the solid particles are hydrophilic for making an oil-in-water emulsion. Thereby, the particles are properly wetted by the continuous phase, i.e. water, that holds the discontinuous phase. The appropriate hydrophilic character may be an inherent characteristic of the solid particles or either enhanced or acquired by treatment of the solid particles.

In the scope of the present invention, "hydrophilic" means that the surface of a corresponding "hydrophilic" solid particle has a contact angle with water against air of < 90°. The contact angle is determined according to methods that are known to the skilled artisan, for example using a standard-instrument (Dropshape Analysis Instrument, Fa. Kruss DAS 10). A shadow image of the droplet is taken using a CCD-camera, and the shape of the droplet is acquired by computer aided image analysis. These measurements are conducted according to DIN 5560-2.

Preferably the droplets that are present in the oil-in-water emulsion have an average droplet size Dv₅₀ in the range of 1 to 40 µm, more preferably in the range of 5 to 40 µm and most preferably in the range of 5 to 30 µm, determined according to ISO13320. Dv₅₀ is defined as the volume median diameter at which 50% of the distribution is contained in droplets that are smal-er than this value while the other half is contained in droplets that are larger than this value.

Preferably the droplets that are present in the oil-in-water emulsion have an average droplet size Dv₉₀ in the range of 40 to 100 µm, more preferably in the range of 40 to 80 µm and most preferably in the range of 40 to 50 µm, determined according to ISO13320. Dv₉₀ is defined as the diameter at which 90% of the distribution is contained in droplets that are smaller than this value while 10% is contained in droplets that are larger than this value.

Preferably the solid particles-stabilized emulsion is free of surfactants. The surfactant can be an anionic, zwitterionic or amphoteric, nonionic or cationic surfactant, or a mixture of two or more of these surfactants. Examples of suitable anionic surfactants include carboxylates, sulfates, sulfonates, phosphonates, and phosphates. Examples of suitable nonionic surfactants include alcohol ethoxylates, alkyl phenol ethoxylates, fatty acid ethoxylates, sorbitan esters and their ethoxylated derivatives, ethoxylated fats and oils, amine ethoxylates, ethylene oxide-propylene oxide copolymers, surfactants derived from mono- and polysaccharides such as the alkyl polyglucosides, and glycerides. Examples of suitable cationic surfactants include quaternary ammonium compounds. Examples of zwitterionic or amphoteric surfactants include N-alkyl betaines or other surfactants derived from betaines.

Preferably the solid particles-stabilized emulsion is free of methyl orange. The molecular structure of methyl orange is as follows:

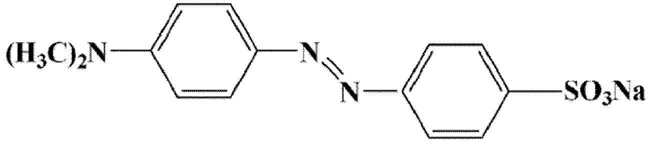

Methyl orange is also known by the IUPAC name sodium 4-[(4-dimethylamino) phenyldiazenyl]benzenesulfonate.

Preferably, the water used for making the solid particles-stabilized emulsion contains ions. Preferably, the total ion concentration is in the range of 3000 to 300000 mg/l, more preferably the total ion concentration is in the range of 100000 to 250000 mg/l, most preferably the total ion concentration is in the range of 200000 to 220000 mg/l.

Preferably the solid particles-stabilized emulsion has a conductivity in the range of 50 to 190 mS/cm, more preferably in the range of 90 to 160 mS/cm.

Preferably the viscosity of the solid particles-stabilized emulsion is in the range of 5 to 30 mPa.s at a temperature of 20 °C under a shear rate of 10/s according to DIN 53019, more preferably in the range of 5 to 20 mPa.s at a temperature of 20 °C under a shear rate of 10/s according to DIN 53019.

The solid particles arrange themselves at positions on the oil/water interface in a manner to prevent droplet coalescence, thus forming a stable emulsion. Preferably the inventively claimed emulsions show a stability of 1 to 30 days at a temperature of in the range of 30-200 °C.

In a preferred embodiment, the presently claimed invention is directed to solid particles-stabilized emulsion containing a continuous phase and a dispersed phase comprising
a) 10 to 90 % by weight water,
b) 10 to 90 % by weight oil and
c) 0.1 to 10 % by weight of at least one layered double hydroxide of general formula (I)

   [M^{II}₍₁₋ₓ₎M^{III}ₓ(OH)₂]^{x+}[Aⁿ⁻]. y H₂O (I),

   wherein
   - M^{II}: denotes a divalent metal ion or 2Li,
   - M^{III}: denotes a trivalent metal ion,
   - Aⁿ⁻: denotes an n-valent anion,
   - n: is 1 or 2,
   - x: is the mole fraction having a value ranging from 0.1 to 0.5 and
   - y: is a value ranging from 0 to 5.0,
   which is present in the form of solid particles,
   wherein water is the continuous phase and oil is the dispersed phase, whereby oil is present in the form of droplets having an average droplet size Dv₅₀ in the range of 1 to 40 µm determined according to ISO13320.

In a more preferred embodiment, the presently claimed invention is directed to solid particles-stabilized emulsion containing a continuous phase and a dispersed phase comprising
a) 50 to 90 % by weight water,
b) 10 to 50 % by weight crude oil and
c) 0.1 to 5 % by weight at least one layered double hydroxide of general formula (I),

   [M^{II}₍₁₋ₓ₎M^{III}ₓ(OH)₂]^{x+}[Aⁿ⁻]. y H₂O (I),

   wherein
   M^{II} denotes a divalent metal ion selected from the group consisting of Ca, Mg, Fe, Ni, Zn, Co, Cu and Mn or 2Li,
   M^{III} denotes a trivalent metal ion selected from the group consisting of Al, Fe, Cr and Mn,
   Aⁿ⁻ denotes an n-valent anion selected from the group consisting of Cl⁻, Br⁻, NO₃-, CO₃²-, SO₄²⁻ and SeO₄²⁻,
   x is the mole fraction having a value ranging from 0.1 to 0.5 any is a value ranging from 0 to 5.0,
   which is present in the form of solid particles,
   wherein water is the continuous phase and oil is the dispersed phase, whereby oil is present in the form of droplets having an average droplet size Dv₅₀ in the range of 5 to 40 µm determined according to ISO13320.

In a most preferred embodiment, the presently claimed invention is directed to solid particles-stabilized emulsion containing a continuous phase and a dispersed phase comprising
a) 50 to 90 % by weight water,
b) 10 to 50 % by weight crude oil and
c) 0.1 to 5 % by weight at least one layered double hydroxide of general formula (I), wherein the layered double hydroxide of general formula (I) is represented by the general formula (I)

   [M^{II}₍₁₋ₓ₎M^{III}ₓ(OH)₂^{]+}[Aⁿ⁻] (I),

   wherein
   M^{II} denotes Mg,
   M^{III} denotes a trivalent metal ion selected from the group consisting of Al and Fe, Aⁿ⁻ denotes an n-valent anion selected from the group consisting of Cl⁻, Br⁻, NO₃-, CO₃²-, SO₄²⁻ and SeO₄²⁻,
   x is the mole fraction having a value ranging from 0.1 to 0.5 and
   y is a value ranging from 0 to 5.0,
   which is present in the form of solid particles,
   wherein water is the continuous phase and oil is the dispersed phase, whereby oil is present in the form of droplets having an average droplet size Dv₅₀ in the range of 5 to 40 µm determined according to ISO13320.

In a further embodiment, the presently claimed invention is directed to a process for the preparation of a solid particles-stabilized emulsion comprising the step of stirring a mixture comprising
a) water, b) oil and c) at least one layered double hydroxide of general formula (I) as define above at a temperature in the range of 30 to 200 °C for a period in the range of 1 min to 2 hours.

Preferably the temperature is in the range of 40 to 150 °C, more preferably in the range of 50 to 100°C.

Preferably the period is in the range of 1 to 90 min, more preferably in the range of 10 to 80 min.

The solid particles are added in an amount that is sufficient to stabilize an oil-in-water emulsion. Preferably, the solid particles are added in an amount of 0.01 to 10 g in relation to 100 ml water, more preferably in amount of 0.01 to 5 g in relation to 100 ml water, most preferably in an amount of 0.01 to 2.5 g in relation to 100 ml water.

The emulsions of the invention can preferably be used in any application for which they are suitable. Examples of such applications include use in cosmetics, drilling for oil and gas, enhanced oil recovery, food, agricultural chemicals, emulsion polymers or latexes, pharmaceuticals, and asphalt emulsions or asphaltic bitumen emulsions. Depending on the use of the emulsion, it can comprise further ingredients, which may either be oil-soluble or water-soluble. For instance, when used in agro formulations, the emulsion suitably contains an agro- chemically active compound. This can be the oil itself or any substance dissolved in the emulsion, such as biocides (including herbicides, fungicides, and pesticides), fertilizers, and the like. Said substance, or each substance when using a combination of substances, can be dissolved in any one of both phases. Similarly, e.g. for cosmetics, the emulsions can contain one or more additional compounds, such as perfumes, vitamins, and the like, dissolved in one or both phases, or as the oil component itself. More preferably the emulsions of the invention are used for enhanced oil recovery.

The preferred embodiments of practicing the invention have been described. It should be understood that the foregoing is illustrative only and that other means and techniques can be employed without departing from the true scope of the invention claimed herein.

### Examples

### Methods

### Emulsion characterization

### -stability

The stability of emulsion was determined by comparing the height of emulsion phases just after forming and after a certain time.

A picture of emulsion was taken with digital camera right after making an emulsion, and after 1 hour, 24 hours, and 1 week. The height of emulsion gradually decreased due to creaming of emulsion phase. Stability of emulsion is defined as a ratio of the height of emulsion phase right after making an emulsion and that of after 24 hours.

### -type

The type of emulsion (oil in water type or water in oil type) was determined by conductivity measurement.

After 24 hours from making an emulsion, the conductivity of emulsion was measured with a conductivity meter (LF330, Wissenschaftlich-Technische Werkstätten GmbH). When conductivity of an emulsion is more than 10 µS / cm, it indicates that the emulsion is oil in water type. When conductivity of an emulsion is less than 10 µS / cm, it indicates that the emulsion is water in oil type (Langmuir 2012, 28, 6769-6775).

### -droplet size

Droplet size of emulsion was measured by the laser diffraction in accordance to ISO13320. The value of Dv₅₀ was used for comparison.

### -viscosity

Viscosity was measured by a rotational viscosity meter at 20 °C and 60 °C in accordance to DIN 53019.

### Temperature and shearing experiment

The stability of emulsion phase under temperature and shearing was determined according to the following procedure: 100 ml of as-made emulsion was poured into a transparent autoclave, and the autoclave was heated to 60 °C and kept for 6 days under continuous stirring (800 U/min).

### Sandpacked column experiments

Flow of the emulsion through porous media, i.e. sandstone or packed sand is essential for practical application. The following experiments allow us to examine the permeability of the obtained emulsion.

A cylinder with height of 200 mm and diameter of 15 mm was used for a vessel. Sand provided by Wintershall (Well: Bockstedt-83) was put into the cylinder until its height be 100 mm. After that, 50 ml of emulsion was poured into the cylinder with 20 ml/min. The amounts of emulsion which went through the sand and droplet size of the emulsion were used as a measure of the ability of the emulsion to flow through the packed column without destruction of the emulsion.

N₂ adsorption desorption isotherms: Langmuir surface areas, BET surface areas, micropore volume, pore volume, micropore size were measured via nitrogen adsorption at 77 K according to DIN 66134 (BET) and DIN 66135 (N₂ adsorption). The micropore volume was determined from the t-plot analysis.

X-ray powder diffraction: The determinations of the crystallinities were performed on a D8 Advance series 2 diffractometer from Bruker AXS. The diffractometer was configured with an opening of the divergence aperture of 0.1 and a Lynxeye detector. The samples were measured in the range from 2 ° to 70 ° (2 Theta). After baseline 30 correction, the reflecting surfaces were determined by making use of the evaluation software EVA (from Bruker AXS). The ratios of the reflecting surfaces are given as percentage values.

### SEM (Method A)

Powder samples were investigated with the field emission scanning electron microscope (FESEM) Hitachi S-4700, which was typically run at acceleration voltages between 2kV and 20kV. Powder samples were prepared on a standard SEM stub and sputter coated with a thin platinum layer, typically 5nm. The sputter coater was the Polaron SC7640. The sizes of LDH particles, diameter and thickness, were counted manually from SEM images. 50 particles were picked up randomly, and their sizes were measured. The averages were defined by the particle sizes. Aspect ratio was determined as the ratio of diameter/thickness.

### Cryo-SEM (Method B)

Aqueous dispersions were investigated with the field emission scanning electron microscope (FESEM) Hitachi S-4700, which was typically run at acceleration voltages between 2kV and 20kV. For the investigation of aqueous dispersions a dedicated cryo equipment from Leica Microsystems is used. Dispersions were shock frozen by immersion in liquid ethane. The frozen hydrated samples were fractured in the MED 020 modular vacuum system fitted with a freeze fracture unit. After freeze etching and Pt sputter coating the frozen samples were transferred with the shuttle VCT100 into the SEM, which is equipped with a cryo-stage. To achieve a high surface sensitivity, avoid beam damage and minimize charging Cryo-SEM imaging was performed at 2kV.

### Elemental analysis

Composition of the obtained materials is measured with flame atomic absorption spectrometry (F-AAS) and inductively coupled plasma optical emission spectrometry (ICP-OES).

### Preparation of layered double hydroxides

### Example 1: synthesis of hydrotalcite

### (Mg²⁺, Al³⁺, CO₃²⁻)

Solution A: Mg(NO₃)₂•6H₂O and Al- (NO₃)₃•9H₂O were dissolved in deionized water (562,5 ml). Solution B: NaOH and Na₂CO₃ were dissolved in deionized water (562,5 ml) to form the mixed base solution. Solution A (562,5 ml) and solution B (562,5 ml) were simultaneously added (5 sec.) under stirring to a vessel containing deionized water (450 ml). The pH of the reaction mixture was around 8.55-8,6. The mixing process was carried out at room temperature. The resulting slurry was transferred to an autoclave and aged at 100 °C for 13 h while stirring (150 U/min). The pH of resulting slurry was 8.38. The slurry was filtered, washed well with 23 L of deionized water, and dried at 120 °C overnight.

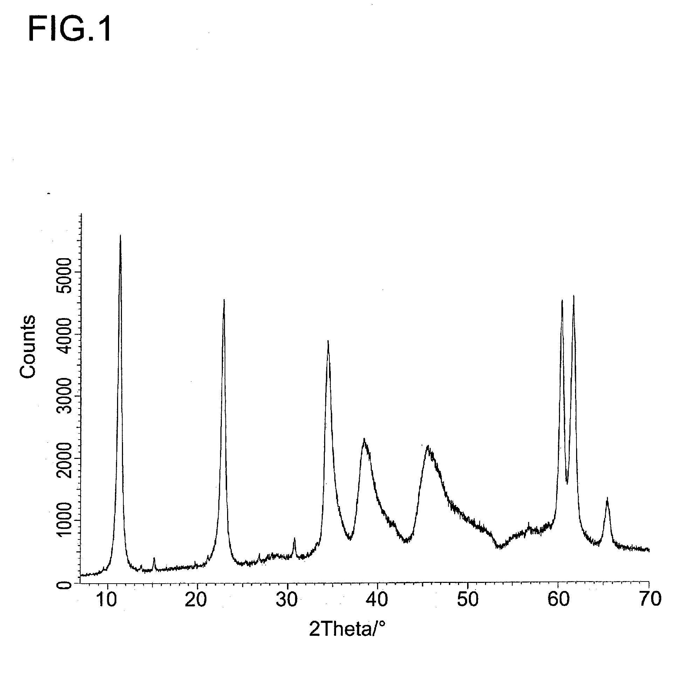

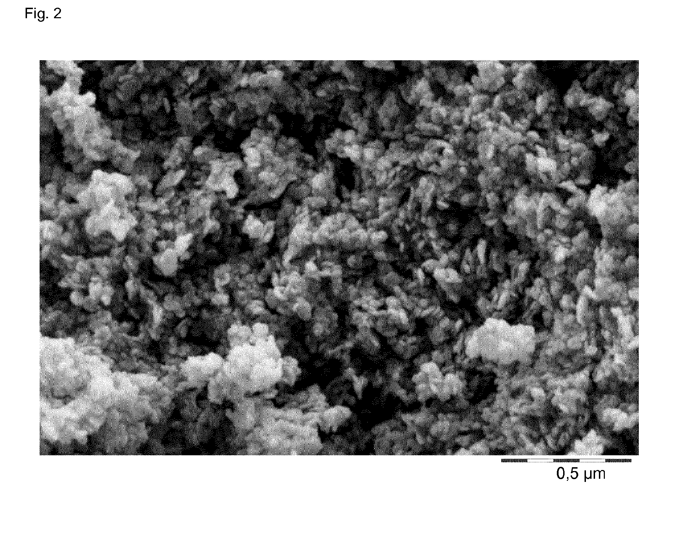

The characterization of the final product by XRD as shown in Figure 1 and table 1 shows that the product has the typical layered double hydroxide structure. The SEM image (Figure 2) shows that the product is a disk shaped material with the diameter of around 50 nm, the thickness of 10-20 nm, and the aspect ratio of 2.5 - 5. The elemental analysis indicated an elemental composition of Mg (23.0 wt. %) and Al (8.2 wt. %). The N₂ adsorption isotherm measurements indicated that the material has BET surface area of 106,3 m²/g.

**Table 1**

| Number | Angle | d-Spacing | Rel. Intensity |
|---|---|---|---|
| 1 | 11.30 | 7.82 | 100% |
| 2 | 15.20 | 5.83 | 3% |
| 3 | 22.82 | 3.89 | 77% |
| 4 | 26.84 | 3.32 | 3% |
| 5 | 30.72 | 2.91 | 5% |
| 6 | 34.43 | 2.60 | 59% |
| 7 | 38.48 | 2.34 | 29% |
| 8 | 45.54 | 1.99 | 26% |
| 9 | 60.36 | 1.53 | 70% |
| 10 | 61.63 | 1.50 | 69% |
| 11 | 65.42 | 1.43 | 12% |

Example 2: synthesis of hydrotalcite-like compound (Mg²⁺, Fe³⁺, CO₃²⁻)Solution A: Mg(NO₃)₂•6H₂O and Fe- (NO₃)₃•9H₂O were dissolved in deionized water (562,5 ml). Solution B: NaOH and Na₂CO₃ were dissolved in deionized water (562,5 ml) to form the mixed base solution. Solution A (562,5 ml) and solution B (562,5 ml) were simultaneously added dropwise to a vessel containing stirred deionized water (450 ml). The pH of the reaction mixture was around 10,6. The mixing process was carried out at room temperature. The resulting slurry was transferred to autoclave and aged at 100 °C for 13 h with 150 U/min stirring. The pH of resulting slurry was 9,5. The slurry was washed well with deionized water with normal filter, and dried at 120 °C overnight.

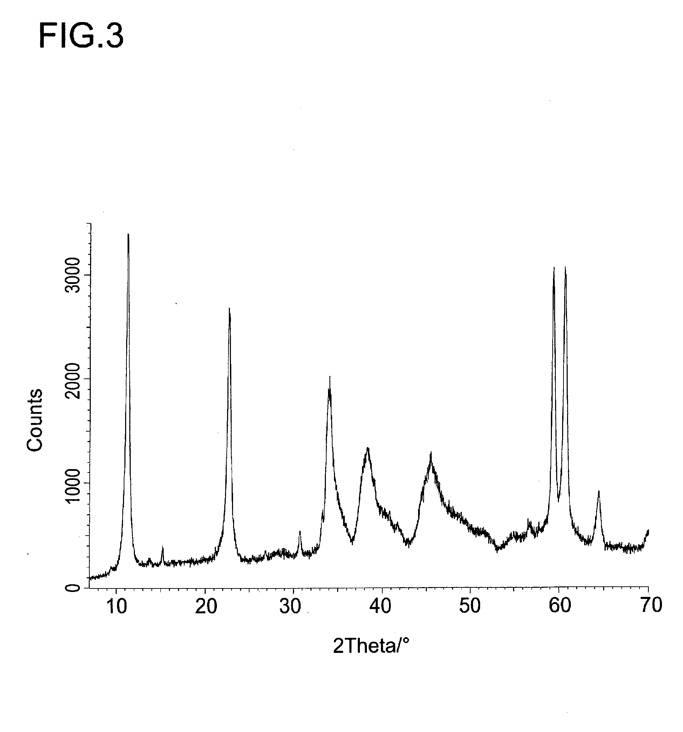

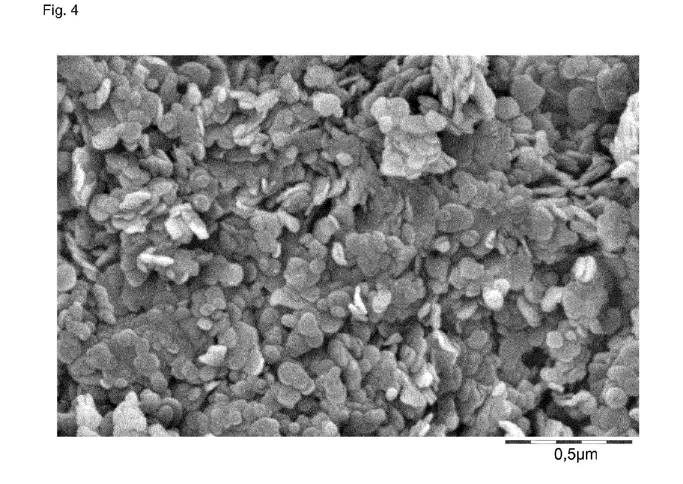

The characterization of the final product by XRD as shown in Figure 3 and table 2 shows that the product has the typical layered double hydroxide structure characteristic. The SEM image (Figure 4) shows that the product is a disk shaped material with the diameter of 30 - 180 nm, the thickness of around 15 nm, and aspect ratio of 2 -12. The elemental analysis indicated an elemental composition of Mg (21,7 wt. %) and Fe (12,6 wt. %). The N₂ adsorption isotherm measurements indicated that the material has BET surface area of 71,0 m²/g.

**Table 2**

| Number | Angle | d-Spacing | Rel. Intensity |
|---|---|---|---|
| 1 | 11.24 | 7.87 | 100% |
| 2 | 15.20 | 5.82 | 6% |
| 3 | 22.67 | 3.92 | 75% |
| 4 | 26.83 | 3.32 | 2% |
| 5 | 30.76 | 2.90 | 7% |
| 6 | 34.00 | 2.63 | 44% |
| 7 | 38.29 | 2.35 | 24% |
| 8 | 45.51 | 1.99 | 20% |
| 9 | 59.38 | 1.56 | 78% |
| 10 | 60.66 | 1.53 | 77% |
| 11 | 64.42 | 1.45 | 15% |

### Comparative example 1: commercial Laponite

Laponite was provided by Rockwood Additives Ltd..

### Preparation of emulsions

For evaluating the obtained materials as emulsifier, emulsion test was performed on the inventive hydrotalcites of example 1 as well as on the commercial laponite. The condition of emulsion test is as follows:
n-undecane (C₁₁H₂₄, Merck, min 99%, 1 L=0,74kg, 1,579 mPa•s @20 °C)
mineral oil (PIONIER 1912, H&R Vertrieb GmbH, 31,4 mPa•s @20 °C)
mineral oil (WIOLTAN SHH 70, H&R Vertrieb GmbH, 222 mPa•s @20 °C)
mineral oil (TUDALEN 900 NF, H&R Vertrieb GmbH , 783,3 mPa•s @20 °C)
crude oil (Wintershall Holding GmbH, 226 mPa•s @20 °C)
x: 0,1, 1, 2,5, 1,0
y:10, 50, 90
z: (100-y) ml
x g of sample and y ml of oil were added to z ml of deionized water. The suspension was heated at 60°C for 1 hour with stirring. After heating, the suspension was stirred with Ultra-turrax with 15*10³ rpm for 3 minutes. Salt water was obtained by dissolving 56429,0 mg of CaCl₂•2H₂O, 22420,2 mg of MgCl₂•6H₂O, 132000,0 mg of NaCl, 270,0 mg of Na₂SO₄, and 380,0 mg of NaBO₂•4H₂O to 1 L of deionized water, adjusting pH to 5,5 - 6,0 with HCl afterwards.

### <emulsion 1>

The compositions of emulsion 1 are as follows: 1g of hydrotalcite (Mg²⁺, Al³⁺, CO₃²⁻), 10 ml of n-undecane (C₁₁H₂₄, Merck, min 99%, 1 L=0,74kg, 1,579 mPa•s @20 °C), and 90 ml of salt water. The stability of the emulsion 1 is 45,9% height after 24 hours. The conductivity of this emulsion was 145 mS / cm which indicates that this emulsion is oil in water type. The results of laser diffraction indicates that this emulsion has Dv₅₀ of 13,1 µm. The viscosity was 8 mPa•s @ 20 °C and 7 mPa•s @ 60°C (under shear rate of 10/s).

### <emulsion 2>

The compositions of emulsion 2 are as follows: 1g of hydrotalcite (Mg²⁺, Al³⁺, CO₃²⁻), 10 ml of mineral oil (PIONIER 1912, H&R Vertrieb GmbH, 31.4 mPa•s @20 °C), and 90 ml of salt water. The stability of the emulsion 2 is 47,2% height after 24 hours. The conductivity of this emulsion was 148 mS / cm which indicates that this emulsion is oil in water type. The results of laser diffraction indicates that this emulsion has Dv₅₀ of 13,6 µm. The viscosity was 10 mPa•s @ 20 °C and 9 mPa•s @ 60 °C (under shear rate of 10/s).

### <emulsion 3>

The compositions of emulsion 3 are as follows: 1g of hydrotalcite (Mg²⁺, Al³⁺, CO₃²⁻), 10 ml of mineral oil (WIOLTAN SHH 70, H&R Vertrieb GmbH , 222 mPa•s @20 °C), and 90 ml of salt water.

The stability of the emulsion 3 is 43,5% height after 24 hours. The conductivity of this emulsion was 151 mS / cm which indicates that this emulsion is oil in water type. The results of laser diffraction indicates that this emulsion has Dv₅₀ of 23,0 µm. The viscosity was 8 mPa•s @ 20 °C and 9 mPa•s @ 60 °C (under shear rate of 10/s).

### <emulsion 4>

The compositions of emulsion 4 are as follows: 1 g of hydrotalcite (Mg²⁺, Al³⁺, CO₃²⁻), 10 ml mineral oil (TUDALEN 900 NF, H&R Vertrieb GmbH , 783,3 mPa•s @20 °C), and 90 ml of salt water.

The stability of the emulsion 4 is 44,3% height after 24 hours. The conductivity of this emulsion was 149 mS / cm which indicates that this emulsion is oil in water type. The results of laser diffraction indicates that this emulsion has Dv₅₀ of 34,4 µm. The viscosity was 10 mPa•s @ 20 °C and 8 mPa•s @ 60 °C (under shear rate of 10/s).

### <emulsion 5>

The compositions of emulsion 5 are as follows: 1g of hydrotalcite (Mg²⁺, Al³⁺, CO₃²⁻), 10 ml of crude oil (Wintershall Holding GmbH, 226 mPa•s @20 °C), and 90 ml of salt water.

The stability of the emulsion 5 is 38,9% height after 24 hours. The conductivity of this emulsion was 152 mS / cm which indicates that this emulsion is oil in water type. The results of laser diffraction indicates that this emulsion has Dv₅₀ of 24,9 µm. The viscosity was 6 mPa•s @ 20 °C and 6 mPa•s @ 60 °C (under shear rate of 10/s).

### <emulsion 6>

The compositions of emulsion 6 are as follows: 1g of hydrotalcite (Mg²⁺, Fe³⁺, CO₃²⁻), 10 ml of mineral oil (PIONIER 1912, H&R Vertrieb GmbH, 31,4 mPa•s @20 °C), and 90 ml of salt water. The stability of the emulsion 6 is 50,9% height after 24 hours. The conductivity of this emulsion was 151 mS / cm which indicates that this emulsion is oil in water type. The results of laser diffraction indicates that this emulsion has Dv₅₀ of 13,7 µm. The viscosity was 20 mPa•s @ 20 °C and 23 mPa•s @ 60 °C (under shear rate of 10/s).

### <emulsion 7 (comparative example)>

The compositions of emulsion 7 are as follows: 1g of commercial laponite, 10 ml of mineral oil (PIONIER 1912, H&R Vertrieb GmbH, 31,4 mPa•s @20 °C), and 90 ml of salt water.

The stability of the emulsion 7 is 29,2 % height after 24 hours. The conductivity of this emulsion was 149 mS / cm which indicates that this emulsion is oil in water type. The results of laser diffraction indicates that this emulsion has Dv₅₀ of 16,1 µm. The viscosity was 88 mPa•s @ 20 °C and 51 mPa•s @ 60 °C (under shear rate of 10/s).

### <emulsion 8 (comparative example)>

The compositions of emulsion 8 are as follows: 1g of commercial laponite, 10 ml of crude oil (Wintershall Holding GmbH, 226 mPa•s @20 °C), and 90 ml of salt water.

The stability of the emulsion 8 is 42,1 % height after 24 hours. The conductivity of this emulsion was 138 mS / cm which indicates that this emulsion is oil in water type. The results of laser diffraction indicates that this emulsion has Dv₅₀ of 26,3 µm. The viscosity was 117 mPa•s @ 20 °C and 73 mPa•s @ 60 °C (under shear rate of 10/s).

## Claims

1. A solid particles-stabilized emulsion containing a continuous phase and a dispersed phase comprising
a) water,
b) oil and
c) at least one layered double hydroxide of general formula (I)
[M^{II}₍₁₋ₓ₎M^{III}ₓ(OH)₂]^{x+}[Aⁿ⁻] .y H₂O (I),
wherein
M^{II} denotes a divalent metal ion or 2Li,
M^{III} denotes a trivalent metal ion,
Aⁿ⁻ denotes an n-valent anion,
n is 1 or 2,
x is the mole fraction having a value ranging from 0.1 to 0.5 and
y is a value ranging from 0 to 5.0, which is present in the form of solid particles,
wherein water is the continuous phase and oil is the dispersed phase, whereby oil is present in the form of droplets.

2. The solid particles-stabilized emulsion according to claim 1, wherein the solid particles-stabilized emulsion comprises 10 to 90 % by weight water, 10 to 90 % by weight oil and 0.1 to 10 % by weight of at least one layered double hydroxide of general formula (I), related to the overall weight of the emulsion.

3. The solid particles-stabilized emulsion according to claim 1 or 2, wherein the oil is crude oil.

4. The solid particles-stabilized emulsion according to one or more of claims 1 to 3, wherein the oil is crude oil having an API gravity in the range between 20 °API and 40 °API.

5. The solid particles stabilized emulsion according to one or more of claims 1 to 4, wherein the oil is crude oil having a viscosity in the range of 1 to 5000 mPa.s at a temperature of 20 °C according to DIN 53019.

6. The solid particles-stabilized emulsion according to claim 1, wherein
the divalent metal ion is Ca, Mg, Fe, Ni, Zn, Co, Cu or Mn,
the trivalent metal ion is Al, Fe, Cr or Mn,
the n-valent anion is Cl⁻, Br⁻,NO₃-, CO₃²-, SO₄²⁻ or SeO₄²⁻,
x is the mole fraction having a value ranging from 0.1 to 0.5 and
y is a value ranging from 0 to 5.0.

7. The solid particles-stabilized emulsion according to one or more of claims 1 to 6, wherein that the droplets have an average droplet size Dv₅₀ in the range of 1 to 40 µm determined according to ISO13320.

8. The solid particles-stabilized emulsion according to one or more of claims 1 to 7, wherein that the droplets have an average droplet size Dv₉₀ in the range of 40 to 100 µm determined according to ISO13320.

9. The solid particles-stabilized emulsion according to one or more of claims 1 to 8, wherein the solid particles have an average particle size in the range of 30 nm to 10 µm determined according to SEM.

10. The solid particles-stabilized emulsion according to one or more of claims 1 to 9, wherein the solid particles-stabilized emulsion is free of surfactants.

11. The solid particles-stabilized emulsion according to one or more of claims 1 to 10, wherein the solid particles-stabilized emulsion is free of methyl orange.

12. The solid particles-stabilized emulsion according to one or more of claims 1 to 11, wherein the solid particles-stabilized emulsion has conductivity in the range of 50 to 190 mS/cm.

13. The solid particles-stabilized emulsion according to one or more of claims 1 to 12, wherein the aspect ratio of the solid particles is in the range of 1 to 30 determined according to SEM images.

14. The solid particles-stabilized emulsion according to one or more of claims 1 to 11, wherein the viscosity of the solid particles-stabilized emulsion is in the range of 5 to 30 mPa.s at a temperature of 20 °C under a shear rate of 10/s according to DIN 53019.

15. A process for the preparation of a solid particles-stabilized emulsion according to one or more of claims 1 to 14, comprising the step of stirring a mixture comprising a) water, b) oil and c) at least one layered double hydroxide of general formula (I) according to one or more of claims 1 to 11 at a temperature in the range of 30 to 200 °C for a period in the range of 1 min to 2 hours.

16. Use of solid particles-stabilized emulsion according to one or more of claims 1 to 14 for enhanced oil recovery.
